# EUROPEAN PATENT SPECIFICATION

(11) **EP 4 687 634 B1**
(45) Date of publication and mention of the grant of the patent: **21.10.2026**
(21) Application number: 24710447.4
(22) Date of filing: 14.03.2024
(51) Int. Cl.: A61B 5/00, A61B 5/271

(54) **PATIENT MONITOR HAVING ONE OR MORE OPTICAL INDICATORS FOR UNIVERSAL PORTS**
PATIENTENMONITOR MIT EINEM ODER MEHREREN OPTISCHEN INDIKATOREN FÜR UNIVERSELLE PORTS
MONITEUR DE PATIENT AYANT UN OU PLUSIEURS INDICATEURS OPTIQUES POUR ORIFICES UNIVERSELS

(30) Priority: 22.03.2023 EP 23163466
(43) Date of publication of application: 11.02.2026
(73) Proprietor: Koninklijke Philips N.V., 5656 AE Eindhoven (NL)
(72) Inventor: EPPING, Christian Michael, 5656 AG Eindhoven (NL); KIM, Sangdo, 5656 AG Eindhoven (NL); ENTERS, Thilo, 5656 AG Eindhoven (NL)
(74) Representative: Philips Intellectual Property & Standards
(86) International application number: PCT/EP2024/056779
(87) International publication number: WO 2024/194133

(56) References cited:
- US-A1- 2013 262 730
- US-A1- 2016 154 942
- US-A1- 2016 183 836
- US-A1- 2018 325 391
- US-A1- 2021 298 991

## Description

### FIELD OF THE INVENTION

The present invention relates to a patient monitor comprising one or more universal ports to connect a cable having a universal connector fitting into an arbitrary universal port.

### BACKGROUND OF THE INVENTION

To acquire a subject's vital signs on a patient monitor, consumables such as electrocardiogram (ECG) lead sets, oxygen saturation (SpO2) sensors, End-tidal CO2 (EtCO2) sensors, invasive blood pressure measurement sensors or blood pressure cuffs are used to interface the patient to the patient monitor. The main function of these consumables is to transmit a measurement signal (e.g., an electrical potential or current) to the patient monitor, where further signal processing is performed in order to determine and display the vital sign.

The consumables can be connected to the patient monitor (or more generally a device) via ports. Conventional consumables each comprise, depending on its type of measurement, its own specific (conventional) connector configured to connect to a corresponding specific (conventional) port of patient monitor. Hence, for each type of measurement, a specific (conventional) port is required to connect the specific (conventional) connector thereto. Recently, there is a trend to use universal ports to connect cables having a universal connector fitting into an arbitrary universal port. The use of universal ports allows to reduce the amount of (different) types of connectors of the consumables and the amount of (different) types of ports of the patient monitor. A universal port supporting not only one but several types of measurements further allows for a more flexible use of the patient monitor.

US 2016/0239623 A1 discloses a medical device comprising a multi-functional socket, the socket capable of being connected with a health information measuring device or a bio sensor module, the type of which are different from each other; and a control portion receiving an identification information from a health information measuring device or a bio sensor module, via the socket, when the socket is connected with the health information measuring device or the bio sensor module, recognizing a type of the health information measuring device or the bio sensor module, and operating an exclusive program for the recognized health information measuring device or the bio sensor module.

US 2016/0183836 A1 discloses a generic activator module that connects to any one of multiple sensor devices via a universal connection port to provide power from a battery to the sensor device and to receive digital physiological data from the sensor device. The generic activator module further has a radio transmitter which transmits the digital physiological data received from the sensor device to a host device.

US 2021/0298991 A1 discloses a medical device that includes one or more removably coupled sensor hubs, a display to provide sensor data, a sensor-agnostic data interface port and a data transfer cable that is compatible with the sensor-agnostic data interface port and includes an electromechanical connector configured to detachably couple to the sensor-agnostic data interface port.

US 2013/0262730 A1 discloses a medical monitoring hub including configurable medical ports and serial ports for communicating with other medical devices in the patient's proximity such as electronic patient bed systems, infusion pump systems or ventilator systems. The hub also includes a docking station for a portable patient monitor, the portable patient monitor communicating with the hub.

### SUMMARY OF THE INVENTION

It is an object of the present invention to provide a patient monitor allowing to use consumables more easily and more flexibly with the patient monitor.

In an aspect a patient monitor is presented that is configured to simultaneously obtain a plurality of measurement signals from a subject and to display one or more of the measurements signals and/or one or more parameters derived therefrom, the patient monitor comprising two or more universal ports each configured to connect a cable having a universal connector fitting into a universal port for providing the measurement signal from the subject to the patient monitor, a detection unit configured to detect a type of cable connected to the universal port, the type of cable relating to a type of measurement; and an optical indicator per universal port, configured to indicate the type of cable connected to the respective universal port wherein the two or more optical indicators are arranged at, around and/or next to the respective universal port and are configured to indicate the type of cable connected to the respective universal port.

The invention is defined in the appended claims.

Preferred embodiments are defined in the dependent claims.

The present invention is based on the findings that with conventional ports, users such as hospital staff may identify the type of cable (relating to the type of consumable and the type of measurement) by looking at the conventional connector. Conventional connectors may differ by characteristics such as size, shape and/or number of pins etc. A user using consumables having a universal connector with universal ports may face the problem of not being able to distinguish different consumables by the type of connector as the universal connectors may look similar externally. This may lead to unfavorable situations for the user when faced with a bundle of cables (of one or more different types), all having a universal connector, and the pressure to act quickly, e.g., during hospital operation.

In particular for hospital operation, hospital staff may require easy, flexible and quick use of consumables with universal ports and/or the patient monitor. For example, the user may be required to attach and/or detach a certain consumable quickly because the course of the patient's treatment may require flexible use of consumables with a specific universal port. For example, some measurements may become dispensable during the patient's treatment and/or other types of measurement may become necessary for further treatment. The user may be thus required to quickly change the type of consumable connected to a specific universal port while avoiding to unintentionally detach another consumable which should not be detached, which in turn could potentially interrupt a currently running measurement.

Interrupting a current measurement may expose the patient to unnecessary health risks or threaten the success of an operation. However, tracing each cable up from the sensor to the universal port connected thereto is inconvenient and time-consuming to determine the type of consumable. Therefore, it is desirable to facilitate the handling of consumables and to enable the user to quickly identify the type of cable (type of consumable, type of measurement) connected with the patient monitor, in particular by looking at a connection section of the patient monitor, the connection section being configured to interface consumables with the one or more universal ports.

Easy attachment (e.g., allowing to check the type of cable of the cable just connected to the universal port) and detachment of consumables to and from the universal port may decrease the stress and workload of the user. Also, the subject's safety may be increased by decreasing the time needed to attach or detach the consumables and by decreasing the risk of mistakes. Delayed start of measurements or mistakes related to the measurements could be harmful or even fatal for the subject. In particular in hospital operation and emergencies, but not limited thereto, the user needs to quickly, easily and safely attach and detach the consumables to ensure a continuous workflow and to move on with other tasks related to the subject's treatment.

The present disclosure provides a patient monitor comprising two or more universal ports.

Different types of cable each relating to a different type of measurement may be used flexibly on the same universal port (one after the other). The patient monitor further comprises a detection unit configured to detect the type of cable connected to the universal port. The patient monitor further comprises two or more optical indicators configured to indicate for each universal port the type of cable of the cable connected thereto and, thus, the type of measurement for which this cable is used. Thus, the patient monitor provided by the present disclosure allows the user to quickly and easily distinguish different cables (consumables, sensors) each having a universal connector and being connected to a universal port. Each universal port may be indicated independently from the other universal ports.

The two or more optical indicators are arranged around, at or next to the two or more universal ports. In particular, an optical indicator may each be arranged around, at and/or next to a respective universal port. Being arranged in the vicinity of or close to the respective universal port allows a user to quickly determine and/or check the type of cable (type of consumable, relating to the type of measurement) since part of the cable, in particular its connector connected to the universal port and the respective optical indicator can be seen at the same time and location.

The optical indicators may e.g., be implemented as light sources emitting light in different colors and/or in a pulsating mode with different or varying pulse lengths. The optical indicators may be arranged on the same face/side of the patient monitor as the two or more universal ports and/or at the connection section of the patient monitor, the connection section comprising the two or more universal ports. Thus, the optical indicators may be in view for a user looking at the two or more universal ports while connecting/removing a universal connector with/from a universal port. In particular, the optical indicators may indicate a respective universal port independently from e.g., a main display of the patient monitor.

It should be noted that the patient monitor may additionally comprise one or more conventional ports (each configured to connect a cable with a non-universal connector, e.g., a (specific) conventional connector with e.g., a certain number of pins). The one or more conventional ports may support core measurements (common and often used types of measurements) such as temperature and blood pressure, and the one or more universal ports may support special measurements. It should be noted, however, that the patient monitor may solely comprise universal ports and/or one or more universal ports supporting core measurements.

The term "patient monitor" refers to a device configured to obtain and process a plurality of measurement signals from a subject. The plurality of measurements signals may be obtained at the same time (simultaneously). Parameters (physiological information) of each of the processed measurement signals, such as heart rate, blood pressure, oxygen saturation or body temperature may be displayed on the patient monitor. Such patient monitors may be used to monitor the condition of the subject, in particular in hospitals, e.g., during intensive care or during a surgery. By looking at the displayed parameters a user, e.g., a clinician, may assess the condition of the subject and decide on the subject's further treatment. The measurement signals may be each obtained via a cable connecting a sensor with the patient monitor. The patient monitor may be connected to a plurality of sensors and thus to a plurality of cables. The patient monitor may be an essentially stationary device located next to the subject, in particular at the bedside, next to a stretcher or next to a chair for subject. The patient monitor may be moveable, e.g., within a room or to another room. For example, the patient monitor may be located on a table with wheels next to a hospital bed.

The term "universal" refers to the capability of the universal port to accept (universal) connectors related to different types of measurements on a subject.

The term "port" refers to a place or section on or at the patient monitor where cables may be connected to via the connector of the cable, wherein the port is configured to obtain electrical information (e.g., a measurement signal, cable information from a memory etc.) from the connected cable.

The term "consumables" (also called "medical consumables") refers to one or more sensors used for a certain type of measurement (e.g., ECG, SpO2, blood pressure etc.) and one or more cables comprising a connector, wherein the one or more sensors and the one or more cables comprising the connector are configured to transmit a measurement signal, e.g., a vital sign obtained from a sensor deployed on a subject for the measurement, to a port of a patient monitor.

The term "type of cable" is linked to the term "type of measurement", i.e. the type of cable relates to a type of measurement and the type of measurement relates to a type of cable. Thus, when the detection unit detects a type of cable, a type of measurement (e.g., ECG, SpO2, etc.) may be detected.

In one embodiment, the patient monitor further comprises a user interface configured to obtain a user request to indicate one or more universal ports, wherein the two or more optical indicators are configured to indicate the one or more universal ports according to the user request. Therefore, a user may require the patient monitor via the user interface to indicate a specific universal port according to the user request with predefined demands.

Preferably, the user interface is configured to obtain a user request to indicate one or more universal ports connected to a cable of one or more requested types of cable, wherein the two or more optical indicators are configured to indicate the one or more universal ports connected to the cable of the one or more requested types of cable. Therefore, each universal port may comprise a respective (corresponding, associated) optical indicator which e.g., emits pulsating light to indicate a requested type of cable. The optical indicator may flash in a particular color or in the color also indicating the type of cable. The patient monitor may also comprise e.g., two optical indicators per universal port, one optical indicator indicating the type of cable and the other optical indicator indicating the universal port connected to the requested type of cable. One optical indicator may also change the color/pulsating mode upon request. Thus, a user may quickly and easily check the type of an attached specific cable and/or quickly and easily detach a specific cable, e.g., to change the type of measurement associated with a specific universal port without running the risk to unintentionally detach another cable which should not be detached at that stage of treatment. This may be useful as some types of measurement are performed several times on the subject such as invasive blood pressure measurement (e.g., arm, leg etc., each location being named differently). In this case, a user required to detach one or more of several cables related to the invasive blood pressure measurement may quickly identify the respective one or more cables (each having a universal connector) while ensuring safe treatment of the subject. Also, the user may check which types of measurements have already been connected to a universal port and whether and which types of measurements are missing.

In another embodiment, the user interface is configured to obtain a user request to indicate one or more universal ports supporting one or more requested types of measurement, wherein the two or more optical indicators are configured to indicate one or more universal ports which support the one or more requested types of measurement. Therefore, a user may quickly determine whether the patient monitor supports all the requested types of measurements and/or which of them. The user may also quickly find the respective universal port for each type of cable. This may reduce the time required to connect a specific cable to a universal port and thus allows for an earlier start of a measurement. This in turn may increase the subject's safety due to the earlier start of measurements and reduce the stress/workload of the user. Also, it may reduce the stress/workload of the user to decide which type of cable should be connected to which universal port. This may be useful if the assignment of several cables to several universal ports may not be done arbitrarily to connect all cables, e.g., if the user must connect several cables and the universal ports of the patient monitor individually support specific types of measurements, wherein some measurements are only supported by certain one or more universal ports.

In a further embodiment, the two or more optical indicators are configured to indicate for each of the two or more universal ports one or more types of measurements supported by the universal port. Thus, the user may quickly determine the capabilities of the patient monitor and/or the two or more universal ports. For each type of cable to be connected, the user may quickly find a respective universal port. This may be useful if the user has to smartly assign several cables of different type to respective universal ports even if the two or more universal ports individually support specific types of measurements and thus for connecting all required cables, the cables may not be plugged arbitrarily into the universal ports. This may be especially useful, if the patient monitor supports only a limited number of specific types of measurement and the universal ports may be flexibly enabled and disabled to support these specific types of measurement. For example, the patient monitor may determine that a maximum number of cables corresponding to this specific type of measurement, e.g., invasive blood measurement, is connected to the patient monitor. The maximum number of supported types of measurement may be given, e.g., by the processing capacities of the patient monitor. The patient monitor may then disable the other universal ports (not connected to this type of cable) to support this type of measurement. The optical indicators corresponding to these other (remaining) universal ports may then indicate that these universal ports do not support invasive blood measurement, although these universal ports may in general support this type of measurement, the support being just temporarily disabled.

The user may therefore not be faced with the problem of not knowing which universal ports do support a certain type of cable or consumable or measurement. The user may otherwise try to connect a type of cable not supported by the universal port or patient monitor, not knowing whether there is a problem which is not related to compatibility, thus potentially wasting time trying to establish a connection or signal transmission between sensor and patient monitor. Loosing precious time could delay the start of a measurement and (further) treatment, which in turn could be harmful or even fatal for the subject. It should be noted that generally, there may be one or more optical indicators each associated to one or more universal ports. Thus, an optical indicator may be e.g., arranged around the respective one or more universal ports and indicate the supported types of measurements for the respective one or more universal ports.

In an embodiment, the detection unit comprises a detection element per universal port arranged within or at the respective universal port, for instance a sensor per universal port to acquire cable type information from the cable inserted into the respective detection element. A universal port and its detection element may thus be manufactured as a unit. In another embodiment, the detection unit comprises a processor or controller, in addition to or instead of the one or more detection elements, to detect the type of cable inserted into a universal port.

In one embodiment, the detection unit is configured to obtain cable information on the type of cable, wherein the cable information is stored in a memory provided at the cable, as an optically readable identification provided at the cable a radio frequency identification (RFID) tag and/or a near field communication (NFC) tag provided in or at the cable. Therefore, the present invention provides several options for the two or more universal ports to obtain the cable information from the cable. The optically readable identification may be a barcode or a quick response (QR) code. The cable information may be also stored in a memory or an NFC tag provided in the connector of the cable. For example, the user may present the cable to the sensor for identification and then plug the cable into the universal port, or the sensor may acquire cable information once the cable has been inserted into the universal port.

In another embodiment, the two or more optical indicators are configured to display different colors, letters and/or graphical symbols. Therefore, the two or more optical indicators may indicate one or more universal ports by displaying e.g., colors and letters according to a coding. A set color coding may be similar or equivalent to the color code used for cables having conventional connectors. For example, the color coding for invasive blood pressure measurements may be red, temperature measurements may be brown and electrocardiogram measurements may be white. The coding may depend on the country, an applicable standard or the application area. For example, universal ports connected to an electrocardiogram (ECG) lead set may be indicated e.g., by the coding according to the Association for the Advancement of Medical Instrumentation (AAMI) standard or the International Electrotechnical Commission (IEC) standard with colors and/or letters. Optical indication of colors may be provided by multicolor LEDs, multiple LEDs of different colors, a light source illuminating different sections of an optical indicator, etc.

In a further embodiment, the two or more optical indicators are configured to indicate whether the type of cable of a cable connected to the respective universal port is supported by the patient monitor and/or the universal port to which the cable is connected. Therefore, the two or more optical indicators may indicate a compatibility and/or a non-compatibility between a type of measurement and a universal port as a confirmation and/or warning to the user. The optical indicator may for example emit blue light for a short time period or permanently during connection as confirmation and pulsating red light as a warning. Thus, a user may acquire immediate feedback from the patient monitor. The two or more optical indicators may also indicate that the universal port connected to a cable relating to a certain type of measurement does not support this type of measurement and immediately indicate one or more other universal ports compatible with this type of measurement, prompting the user to use the suggested universal port. It should be noted that one universal port may have several associated optical indicators. The feedback may be issued by the same optical indicator indicating the type of cable but also by another optical indicator associated with the same universal port.

In one embodiment, the two or more optical indicators are configured to indicate a status of a measurement on a subject; a status of a transmission of a measurement signal; parameters of a measurement; and/or parameters of a measurement on a subject being in or outside a valid range of the parameters. Thus, the user may quickly assess details of the measurement and/or transmission and the progress of the measurement and/or transmission of the measurement signal. The two or more optical indicators may indicate the progress of a measurement, whether a cable is (correctly) connected to a universal port or loosened itself, the transmission of a measurement signal being active, etc. The user may also quickly assess parameters of a measurement and/or whether a parameter of the measurement is within or outside of a valid range of the parameter, requiring further steps in the treatment of the subject. For example, one or more optical indicators may indicate a pulse of the patient with flashing and an oxygen saturation with color saturation or light saturation. Thus, the user may assess details of the performed measurements even if the user has a restricted view onto a main display of the patient monitor (displaying the processed measurement signals in detail) while treating the subject (e.g., patient) from a certain position relative to the patient monitor in a room or with other users blocking the view onto the main display of the patient monitor.

In another embodiment, the two or more optical indicators are configurable according to user preferences. Therefore, a user may change the settings of the two or more optical indicators according to specific demands of a user such as a color coding being common in the user's country or habits in a hospital, specific parameters, etc.

In a further embodiment, the patient monitor further comprises one or more further optical indicators per universal port. Therefore, the patient monitor may comprise several optical indicators per universal port, wherein the optical indicators associated with the universal port are each configured to indicate in a differently manner (e.g., different colors, letters, symbols, etc.) and/or different information. Thus, for each universal port, respective optical indicators may be each associated e.g., to a certain type of cable or type of measurement, wherein each optical indicator is configured to e.g., emit one color only.

In one embodiment, one or more optical indicators are arranged as a group at and/or next to a group of one or more universal ports. An assignment of the one or more optical indicators with the one or more associated (corresponding) universal ports may allow the user to (quickly) identify the one or more universal ports associated with an optical indicator. The assignment may be made via e.g., (system of) numbering or an (optical, geometrical) arrangement of the group.

In another embodiment, one or more optical indicators are displays. The displays may indicate colors, letters and/or graphical symbols. Preferably, the display is an electronic ink display. It should be noted that in other embodiments, the display may be of other types. It should be also noted that the patient monitor may comprise several types of optical indicators such as LEDs and electronic ink displays or different types of displays. For example, the patient monitor may comprise one or more LEDs at each universal port and a display next to the universal ports, or the optical indicator may be configured as a ring around the respective universal port.

In a further embodiment, one or more universal ports are arranged on or at a plug-in module configured to be plugged into the patient monitor. Therefore, the capabilities of (the types of measurements supported by) the patient monitor may be flexibly changed by adding, exchanging and/or removing plug-in modules each comprising one or more universal ports. Thus, universal ports supporting certain types of measurements may be flexibly used on one or several patient monitors.

### BRIEF DESCRIPTION OF THE DRAWINGS

These and other aspects will be apparent from and elucidated with reference to the embodiment(s) described hereinafter. In the following drawings:
Fig. 1 shows a schematic diagram illustrating a measurement on a subject according to an embodiment.
Fig. 2A shows a schematic diagram of a conventional patient monitor comprising conventional ports.
Fig. 2B shows a schematic diagram of a first embodiment of a patient monitor
Fig. 2C shows a schematic diagram of a second embodiment of a patient monitor having multiple detection elements.
Fig. 3 shows a schematic diagram of a third embodiment of a patient monitor comprising a user interface.
Fig. 4 shows a schematic diagram of a fourth embodiment of a patient monitor
Fig. 5 shows a schematic diagram of a fifth embodiment of a patient monitor
Fig. 6 shows a schematic diagram of a sixth embodiment of a patient monitor comprising a plug-in module.

### DETAILED DESCRIPTION OF EMBODIMENTS

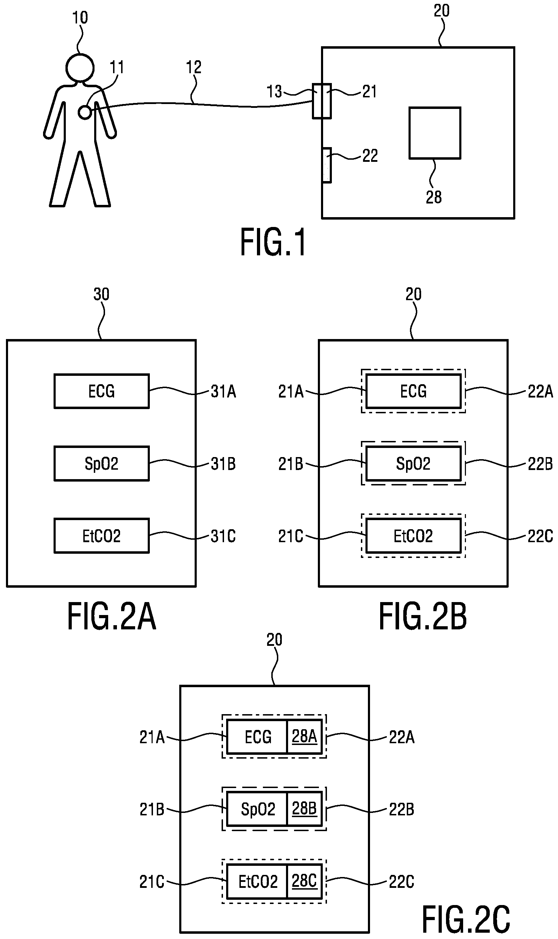

Fig. 1 schematically shows a diagram illustrating a measurement on a subject 10 with a patient monitor 20 according to an embodiment.

A sensor 11 (such as an electrocardiogram (ECG) lead set, an oxygen saturation (SpO2) sensor or an End-tidal CO2 (EtCO2) sensor, an invasive blood pressure sensor, a non-invasive blood pressure cuff etc.) is deployed on the subject 10 for acquiring/obtaining a measurement signal. A cable 12 is configured to transmit the measurement signal from sensor 11 to patient monitor 20 via a universal connector 13 and a universal port 21. The measurement signal (e.g., a vital sign or physiological signal) may then be processed by the patient monitor 20 and displayed on the patient monitor 20. A detection unit 28 (e.g., a controller or processor) is configured to detect a type of cable (relating to the type of consumable (i.e., a sensor and cable with a connector) and the type of measurement such as ECG, EtCO2 or blood pressure etc.). The detection unit 28 may e.g., detect the type of cable based on cable information acquired by a sensor configured to acquire cable information stored within or at the cable, or the detection unit 28 may detect the type of cable based on characteristic of the measurement signal itself. The patient monitor 20 shown in Fig. 1 further comprises an optical indicator 22 configured to indicate the detected type of cable of cable 12 connected to the universal port 21. A user may therefore recognize the type of cable connected to the universal port 21 by looking at the optical indicator 22 associated with and arranged near the universal port 21.

It should be noted that patient monitor 20 may generally comprise any number of universal ports 21, i.e., one or more universal ports 21, and any number of optical indicators 22, i.e., one (or more) optical indicator(s) 22 per universal port.

Fig. 2A shows a schematic diagram of a conventional patient monitor 30 with three conventional ports 31A, 31B, 31C. Conventional ports 31A, 31B, 31C are each configured to connect to a specific conventional connector connected via a cable to a specific sensor. In Fig. 2A, the first conventional port 31A is connected to a cable having a conventional connector of an ECG sensor. The second conventional port 31B is connected to a cable having a conventional connector of an SpO2 sensor. The third conventional port 31C is connected to a cable having a conventional connector of an EtCO2 sensor.

These conventional connectors may differ regarding their characteristics such as size, shape and/or number of pins, allowing to distinguish the type of conventional connectors by their outer appearance. For each type of measurement, the conventional connector must be connected with a specific conventional port 31A, 31B, 31C in order to establish transmission of the measurement signal to the (conventional) patient monitor 30. Conventional connectors may comprise the function of a Poka Yoke element, in the sense that a user may quickly recognize by its characteristics whether the conventional connector may be connected to a specific conventional port 31A, 31B, 31C - a conventional connector of an electrocardiogram sensor may not be plugged into a conventional port supporting blood pressure measurement. However, the use of conventional ports results in a variety of conventional connectors and a restricted flexibility in conducting measurements as available measurements are restricted due to the limited space for ports in a patient monitor.

Fig. 2B shows a schematic diagram of an embodiment of a patient monitor 20 comprising three universal ports 21A, 21B, 21C according to the present invention. In contrast to conventional ports 31A, 31B, 31C shown in Fig. 2A, each universal port 21A, 21B, 21C may connect to different types of cable (different types of consumables, relating to different types of sensors/measurements). Each universal port 21A, 21B, 21C is configured to connect different cables 12 having a universal connector 13 fitting into the universal port 21A, 21B, 21C. In Fig. 2B, universal port 21A is connected to a cable 12 connected to an ECG lead set via a universal connector 13, universal port 21B is connected to another cable 12 connected to an SpO2 sensor via another universal connector 13, and universal port 21C is connected to a further cable 12 connected to an EtCO2 sensor via a further universal connector 13. Each universal port 21A, 21B, 21C supports several types of measurements, i.e. universal port 21A may also connect to the SpO2 sensor or the EtCO2 sensor. However, a user using different types of cable with the patient monitor 20 may not be able to distinguish cables 12 by the outer appearance of the universal connectors 13. The cables themselves may not (or not easily) allow to recognize the type of measurement performed by use of a particular cable as well.

In the embodiment shown in Fig. 2B, the user may determine the type of cable connected to and/or supported by the universal port 21A, 21B, 21C by their associated optical indicators 22A, 22B, 22C. In this embodiment, the patient monitor 20 comprises three optical indicators 22A, 22B, 22C, each arranged around a respective universal port 21A, 21B, 21C. Optical indicator 22A is arranged around universal port 21A, optical indicator 22B is arranged around universal port 21B and optical indicator 22C is arranged around universal port 21C. The optical indicators 22A, 22B, 22C indicate for each universal port 21A, 21B, 21C the type of the cable connected to and/or supported by each universal port 21A, 21B, 21C. Supported types of cable may be indicated e.g., by standard or upon/according to a user request. As a non-limiting example, optical indicator 22A may emit green light, optical indicator 22B may emit red light and optical indicator 22C may emit blue light (the colors being indicated by different lines in Fig. 2B). Generally, a predetermined color coding may be applied to indicate the different types of cable. Further, more generally, the optical indicators 22A, 22B, 22C may operate in different modes such as a pulsating mode (flashing in constant or varying time intervals), a constant mode, in different colors, different widths or portions of sections of the optical indicator 22A, 22B, 22C illuminated, etc. The optical indicators 22A, 22B and 22C may be controlled by a controller.

It should be noted that patient monitor 20 may additionally comprise any number of conventional ports (i.e., none, one or more). Providing both universal ports and conventional ports may facilitate a transition from conventional ports to universal ports by supporting e.g., hospitals refraining from making the complete change for the time being so that some of the conventional cables may be used as well. Further, some standard measurements that are always made (e.g., SpO2, ECG, etc.) may be made by standard cables that can thus be connected to conventional ports.

Fig. 2C shows another schematic diagram of an embodiment of the patient monitor 20 comprising three universal ports 21A, 21B, 21C.

According to this embodiment, three detection elements 28A, 28B, 28C are arranged at respective universal ports 21A, 21B, 21C. The detection elements 28A, 28B, 28C may each comprise a sensor configured to detect the type of cable connected to the respective universal port 21A, 21B, 21C. For example, the user may present, e.g., a barcode of the cable to the sensor before plugging the cable into the respective universal port 21A, 21B, 21C. Alternatively, the sensor may read the cable information when the cable is already plugged into the universal port 21A, 21B, 21C. This may ensure that for each universal port 21A, 21B, 21C only the cable information of the cable connected thereto is read and thus prevent mix-ups, e.g., by the user presenting a cable to the detection element 28A and then plugging the cable into the universal port 28B.

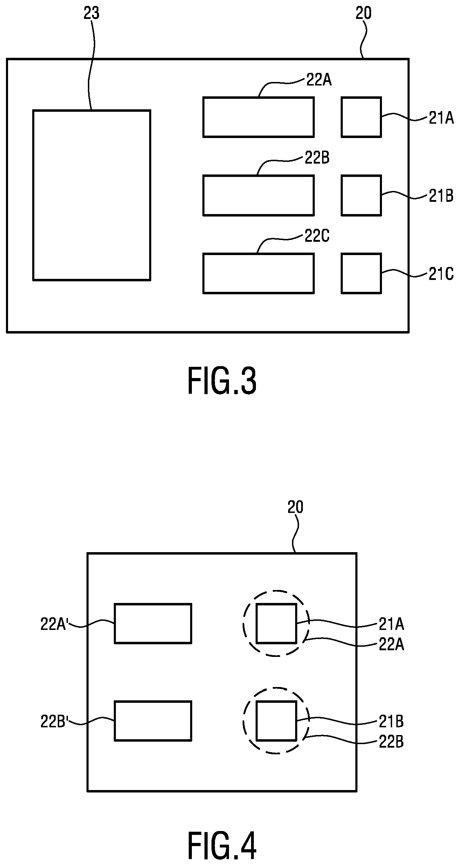

Fig. 3 shows a schematic diagram of another embodiment of a patient monitor 20 according to the present invention comprising a user interface 23, three universal ports 21A, 21B, 21C and three optical indicators 22A, 22B, 22C. The user interface 23 may obtain and process a user request and cause the optical indicators 22A, 22B, 22C to indicate one or more of the universal ports 21A, 21B, 21C according to the user request, i.e., to make it visible and easily recognizable for a user which of the universal ports shall be used or is used for a certain measurement. The user may e.g., request to indicate one or more universal ports supporting e.g., blood pressure measurement and/or a universal port connected to a certain type of cable, e.g., relating to an oxygen saturation measurement or an invasive blood pressure measurement applicated to the left leg of the subject.

In an embodiment the optical indicators 22A, 22B, 22C may be electronic ink displays that are able to indicate the types of cable of cables 12 connected to the respective universal ports 21A, 21B, 21C according to a letter coding. The optical indicators 22A, 22B, 22C may hereby be arranged at (e.g., directly adjacent to) the universal ports 21A, 21B, 21C.

The user interface 23 may be arranged at the connecting section comprising the optical indicators 22A, 22B, 22C next to the universal ports 21A, 21B, 21C but may also be arranged anywhere at the patient monitor 20, e.g., integrated into a main display of the patient monitor which displays processed measurement signals.

Fig. 4 shows a schematic diagram of another embodiment of a patient monitor 20 according to the present invention comprising two universal ports 21A, 21B and, for each universal port 21A, 21B, two associated optical indicators. For each universal port 21A, 21B one (associated) optical indicator 22A, 22B is arranged around the universal port and the other (associated) optical indicator 22A', 22B' is arranged next to the (associated) universal port 21A, 21B. In the embodiment shown in Fig. 4, optical indicators 22A and 22A' are associated with universal port 21A and optical indicators 22B and 22B' are associated with universal port 21B.

Optical indicators 22A, 22B may be multicolor LEDs each configured to indicate the type of cable connected to the universal port 21A, 21B by a color according to a color code. Optical indicators 22A, 22B may additionally indicate the compatibility of the type of cable with the universal port 21A, 21B (e.g., flashing red light to indicate non-compatibility to a user and blue light for a short time period to indicate compatibility). Optical indicators 22A', 22B' may indicate the types of measurements (types of cable/consumable/measurement) supported by the universal port 21A, 21B and/or parameters of a measurement. The optical indicators 22A, 22A', 22B, 22B' may also be arranged partially around universal ports 21A, 21B and/or (directly adjacent) at the universal ports 21A, 21B.

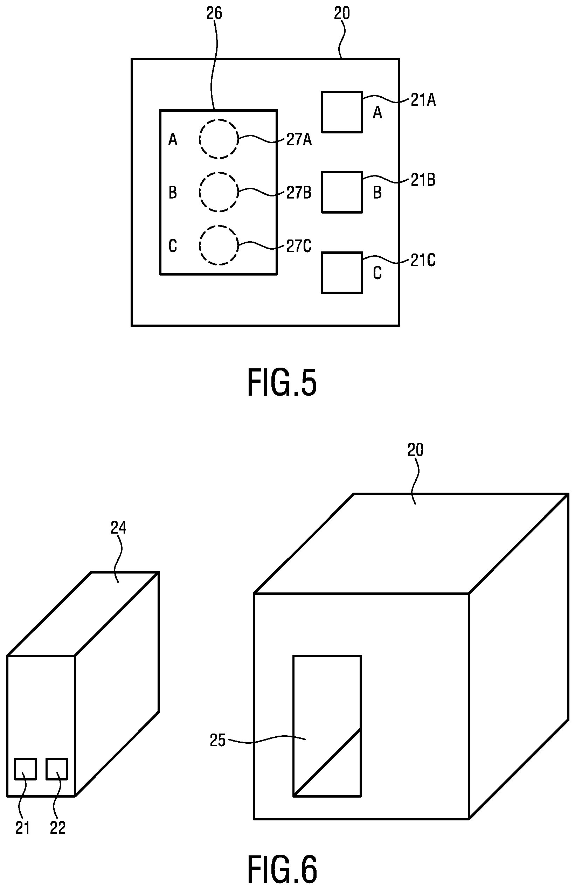

Fig. 5 shows a schematic diagram of another embodiment of a patient monitor 20 according to the present invention. The patient monitor 20 according to this embodiment comprises three universal ports 21A, 21B, 21C and an optical indication unit 26. The optical indication unit 26 may e.g., be implemented as a display or touchscreen and comprise various sections 27A, 27B, 27C representing optical indicators that indicate each universal port 21A, 21B, 21C on an assigned section. In the embodiment shown in Fig. 4, the sections 27A, 27B, 27C of the optical indication unit 22 are marked by the letters A, B and C which each correspond to a universal port 21A, 21B, 21C marked by the corresponding letter so that the user can easily recognize to which universal port the respective optical indicator belongs.

Fig. 6 shows a schematic diagram of a plug-in module 24 comprising a universal port 21 and an optical indicator 22 according to another embodiment.

The patient monitor 20 comprises a receptacle 25 configured to receive the plug-in module 24. The plug-in module 24 may be inserted into patient monitor 20, exchanged by another plug-in module 24 or removed from patient monitor 20. One plug-in module 24 (universal port) may support several certain types of measurements, allowing for more flexible measurements using the patient monitor 20 by being exchangeable and/or broadening the supported types of measurements of a sole patient monitor 20. The plug-in module 24 may comprise one or more universal ports 21 and/or one or more optical indicators 22. Plug-in module 24 may also additionally comprise one or more conventional ports 31.

In summary, the present invention may provide a user-friendly solution to use consumables having a universal connector more easily and more flexible with two or more universal ports of a connecting device such as a patient monitor. In particular, the present invention may allow a user to quickly identify cables having a universal connector.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. A patient monitor (20) configured to simultaneously obtain a plurality of measurement signals from a subject (10) and to display one or more of the measurements signals and/or one or more parameters derived therefrom, the patient monitor (20) comprising:
two or more universal ports (21) each configured to connect a cable (12) having a universal connector (13) fitting into a universal port (21) for providing the measurement signal from the subject (10) to the patient monitor (20);
a detection unit (28; 28A-28C) configured to detect a type of cable connected to a universal port (21), the type of cable relating to a type of measurement; and
an optical indicator (22) per universal port (21), configured to indicate the type of cable connected to the respective universal port (21), wherein the two or more optical indicators (22) are arranged at, around and/or next to the respective universal port (21) and are configured to indicate the type of cable connected to the respective universal port (21),
wherein the patient monitor supports a limited number of specific one or more types of measurement and the two or more universal ports (21) are flexibly enabled and disabled to support these types of measurement; and
wherein the two or more optical indicators (22) are configured to indicate for each of the two or more universal ports (21) the one or more types of measurement supported by the universal ports (21).

2. Patient monitor (20) as claimed in claim **1,**
further comprising a user interface (23) configured to obtain a user request to indicate one or more universal ports (21), wherein the two or more optical indicators (22) are configured to indicate the one or more universal ports (21) according to the user request.

3. Patient monitor (20) as claimed in claim 2,
wherein the user interface (23) is configured to obtain a user request to indicate one or more universal ports (21) connected to a cable (12) of one or more requested types of cable,
wherein the two or more optical indicators (22) are configured to indicate the one or more universal ports (21) connected to the cable (12) of the one or more requested types of cable.

4. Patient monitor (20) as claimed in claim 2 or 3,
wherein the user interface (23) is configured to obtain a user request to indicate one or more universal ports (21) supporting one or more requested types of measurement,
wherein the two or more optical indicators (22) are configured to indicate one or more universal ports (21) which support the one or more requested types of measurement.

5. Patient monitor (20) as claimed in any one of the preceding claims,
wherein the detection unit comprises one or more of:
- a detection element (28A-28C) per universal port (21A-21C) arranged within or at the respective universal port; and
- a processor or controller (28).

6. Patient monitor (20) as claimed in any one of the preceding claims,
wherein the detection unit (28; 28A-28C) is configured to obtain cable information on the type of cable, wherein the cable information is stored:
in a memory provided at the cable (12);
as an optically readable identification provided at the cable (12); and/or
a radio frequency identification, RFID, tag or a near field communication, NFC, tag provided in or at the cable (12).

7. Patient monitor (20) as claimed in any one of the preceding claims,
wherein the two or more optical indicators (22) are configured to display different colors, letters and/or graphical symbols.

8. Patient monitor (20) as claimed in any one of the preceding claims,
wherein the two or more optical indicators (22) are configured to indicate whether the type of cable of a cable (12) connected to the respective universal port (21) is supported by the patient monitor (20) and/or the universal port (21) to which the cable (12) is connected.

9. Patient monitor (20) as claimed in any one of the preceding claims,
wherein the two or more optical indicators (22) are configured to indicate:
a status of a measurement on a subject (10);
a status of a transmission of a measurement signal;
parameters of a measurement; and/or
parameters of a measurement on a subject (10) being in or outside a valid range of the parameters.

10. Patient monitor (20) as claimed in any one of the preceding claims,
wherein the two or more optical indicators (22) are configurable according to user preferences.

11. Patient monitor (20) as claimed in any one of the preceding claims,
further comprising one or more further optical indicators (22) per universal port (21).

12. Patient monitor (20) as claimed in any one of the preceding claims,
wherein one or more optical indicators (22) are arranged as a group at and/or next to a group of one or more universal ports (21).

13. Patient monitor (20) as claimed in any one of the preceding claims,
wherein one or more optical indicators (22) are displays, in particular electronic ink displays.

14. Patient monitor (20) as claimed in any one of the preceding claims,
wherein one or more universal ports (21) are arranged on or at a plug-in module (24) configured to be plugged into the patient monitor (20).

## Patentansprüche

1. Patientenmonitor (20), der konfiguriert ist, um gleichzeitig eine Vielzahl von Messsignalen von einer Zielperson (10) zu erhalten und eines oder mehrere der Messsignale und/oder einen oder mehrere daraus abgeleitete Parameter anzuzeigen, wobei der Patientenmonitor (20) Folgendes umfasst:
zwei oder mehr Universalanschlüsse (21), die jeweils konfiguriert sind, um ein Kabel (12) mit einem Universalstecker (13), der in einen Universalanschluss (21) passt, anzuschließen, um dem Patientenmonitor (20) das Messsignal von der Zielperson (10) bereitzustellen;
eine Detektionseinheit (28; 28A-28C), die konfiguriert ist, um einen an einen Universalanschluss (21) angeschlossenen Kabeltyp zu detektieren, wobei der Kabeltyp einem Messtyp zugeordnet ist; und
einen optischen Indikator (22) pro Universalanschluss (21), der konfiguriert ist, um den an den jeweiligen Universalanschluss (21) angeschlossenen Kabeltyp anzugeben, wobei die zwei oder mehr optischen Indikatoren (22) an, um und/oder neben dem jeweiligen Universalanschluss (21) angeordnet sind und konfiguriert sind, um den an den jeweiligen Universalanschluss (21) angeschlossenen Kabeltyp anzugeben,
wobei der Patientenmonitor eine begrenzte Anzahl eines oder mehrerer spezifischer Messtypen unterstützt und die zwei oder mehr Universalanschlüsse (21) flexibel aktiviert und deaktiviert werden können, um diese Messtypen zu unterstützen; und
wobei die zwei oder mehr optischen Indikatoren (22) konfiguriert sind, um für jeden der zwei oder mehr Universalanschlüsse (21) den einen oder die mehreren von den Universalanschlüssen (21) unterstützten Messtypen anzugeben.

2. Patientenmonitor (20) nach Anspruch 1,
weiter umfassend eine Benutzerschnittstelle (23), die konfiguriert ist, um eine Benutzeranforderung zu erhalten, um einen oder mehrere Universalanschlüsse (21) anzugeben, wobei die zwei oder mehr optischen Indikatoren (22) konfiguriert sind, um den einen oder die mehreren Universalanschlüsse (21) gemäß der Benutzeranforderung anzugeben.

3. Patientenmonitor (20) nach Anspruch 2,
wobei die Benutzerschnittstelle (23) konfiguriert ist, um eine Benutzeranforderung zu erhalten, um einen oder mehrere Universalanschlüsse (21) anzugeben, die an ein Kabel (12) eines oder mehrerer angeforderter Kabeltypen angeschlossen sind,
wobei die zwei oder mehr optischen Indikatoren (22) konfiguriert sind, um den einen oder die mehrere Universalanschlüsse (21) anzugeben, die an das Kabel (12) des einen oder der mehreren angeforderten Kabeltypen angeschlossen sind.

4. Patientenmonitor (20) nach Anspruch 2 oder 3,
wobei die Benutzerschnittstelle (23) konfiguriert ist, um eine Benutzeranforderung zu erhalten, um einen oder mehrere Universalanschlüsse (21) anzugeben, die einen oder mehrere angeforderte Messtypen unterstützen,
wobei die zwei oder mehr optischen Indikatoren (22) konfiguriert sind, um einen oder mehrere Universalanschlüsse (21) anzugeben, die den einen oder die mehreren angeforderten Messtypen unterstützen.

5. Patientenmonitor (20) nach einem der vorstehenden Ansprüche,
wobei die Detektionseinheit eines oder mehrere von Folgendem umfasst:
- ein Detektionselement (28A-28C) pro Universalanschluss (21A-21C), das innerhalb oder an dem jeweiligen Universalanschluss angeordnet ist; und
- einen Prozessor oder eine Steuereinheit (28).

6. Patientenmonitor (20) nach einem der vorstehenden Ansprüche,
wobei die Detektionseinheit (28; 28A-28C) konfiguriert ist, um Kabelinformationen über den Kabeltyp zu erhalten, wobei die Kabelinformationen gespeichert werden:
in einem an dem Kabel bereitgestellten Speicher (12);
als eine optisch lesbare Identifikation, die an dem Kabel (12) bereitgestellt ist; und/oder
ein Radiofrequenzidentifikations-Tag (RFID-Tag) oder ein Nahfeldkommunikations-Tag (NFC-Tag), das in oder an dem Kabel (12) bereitgestellt ist.

7. Patientenmonitor (20) nach einem der vorstehenden Ansprüche,
wobei die zwei oder mehr optischen Indikatoren (22) konfiguriert sind, um unterschiedliche Farben, Buchstaben und/oder grafische Symbole anzuzeigen.

8. Patientenmonitor (20) nach einem der vorstehenden Ansprüche,
wobei die zwei oder mehr optischen Indikatoren (22) konfiguriert sind, um anzugeben, ob der Kabeltyp eines an den jeweiligen Universalanschluss (21) angeschlossenen Kabels (12) von dem Patientenmonitor (20) und/oder dem Universalanschluss (21), an den das Kabel (12) angeschlossen ist, unterstützt wird.

9. Patientenmonitor (20) nach einem der vorstehenden Ansprüche,
wobei die zwei oder mehr optischen Indikatoren (22) konfiguriert sind, um Folgendes anzugeben:
einen Status einer Messung an einer Zielperson (10);
einen Status einer Übertragung eines Messsignals;
Parameter einer Messung; und/oder
Parameter einer Messung an einer Zielperson (10), die in oder außerhalb eines gültigen Bereichs der Parameter liegen.

10. Patientenmonitor (20) nach einem der vorstehenden Ansprüche,
wobei die zwei oder mehr optischen Indikatoren (22) gemäß den Vorlieben des Benutzers konfigurierbar sind.

11. Patientenmonitor (20) nach einem der vorstehenden Ansprüche,
weiter umfassend einen oder mehrere weitere optische Indikatoren (22) pro Universalanschluss (21).

12. Patientenmonitor (20) nach einem der vorstehenden Ansprüche,
wobei ein oder mehrere optische Indikatoren (22) als eine Gruppe an und/oder neben einer Gruppe von einem oder mehreren Universalanschlüssen (21) angeordnet sind.

13. Patientenmonitor (20) nach einem der vorstehenden Ansprüche,
wobei einer oder mehreren optischen Indikatoren (22) Anzeigen sind, insbesondere Anzeigen mit elektronischer Tinte.

14. Patientenmonitor (20) nach einem der vorstehenden Ansprüche,
wobei ein oder mehrere Universalanschlüsse (21) auf oder an einem Steckmodul (24) angeordnet sind, das zum Einstecken in den Patientenmonitor (20) konfiguriert ist.

## Revendications

1. Moniteur patient (20) configuré pour obtenir simultanément une pluralité de signaux de mesure provenant d'un sujet (10) et pour afficher un ou plusieurs de ces signaux de mesure et/ou un ou plusieurs paramètres qui en sont dérivés, le moniteur patient (20) comprenant :
deux ou plusieurs ports universels (21), chacun configuré pour connecter un câble (12) doté d'un connecteur universel (13) s'insérant dans un port universel (21) pour fournir le signal de mesure du sujet (10) au moniteur patient (20) ;
une unité de détection (28 ; 28A-28C) configurée pour détecter un type de câble connecté à un port universel (21), le type de câble étant lié à un type de mesure ; et
un indicateur optique (22) par port universel (21), configuré pour indiquer le type de câble connecté au port universel (21) correspondant, dans lequel les deux ou plusieurs indicateurs optiques (22) sont agencés autour et/ou à côté du port universel respectif (21) et sont configurés pour indiquer le type de câble connecté au port universel respectif (21),
dans lequel le moniteur patient prend en charge un nombre limité d'un ou plusieurs types de mesures spécifiques et les deux ou plusieurs ports universels (21) sont activés et désactivés de manière flexible pour prendre en charge ces types de mesures ; et
dans lequel les deux ou plusieurs indicateurs optiques (22) sont configurés pour indiquer, pour chacun des deux ou plusieurs ports universels (21), le ou les types de mesure pris en charge par les ports universels (21).

2. Moniteur patient (20) selon la revendication 1,
comprenant en outre une interface utilisateur (23) configurée pour obtenir une demande de l'utilisateur afin d'indiquer un ou plusieurs ports universels (21), dans lequel les deux ou plusieurs indicateurs optiques (22) sont configurés pour indiquer le ou les ports universels (21) selon la demande de l'utilisateur.

3. Moniteur patient (20) selon la revendication 2,
dans lequel l'interface utilisateur (23) est configurée pour obtenir une demande de l'utilisateur afin d'indiquer un ou plusieurs ports universels (21) connectés à un câble (12) d'un ou plusieurs types de câbles demandés,
dans lequel les deux ou plusieurs indicateurs optiques (22) sont configurés pour indiquer les un ou plusieurs ports universels (21) connectés au câble (12) du ou des types de câbles demandés.

4. Moniteur patient (20) selon la revendication 2 ou 3,
dans lequel l'interface utilisateur (23) est configurée pour obtenir une demande de l'utilisateur indiquant un ou plusieurs ports universels (21) prenant en charge un ou plusieurs types de mesure demandés,
dans lequel les deux ou plusieurs indicateurs optiques (22) sont configurés pour indiquer un ou plusieurs ports universels (21) qui prennent en charge le ou les types de mesure demandés.

5. Moniteur patient (20) selon l'une quelconque des revendications précédentes,
dans lequel l'unité de détection comprend un ou plusieurs parmi :
- un élément de détection (28A-28C) par port universel (21A-21C) agencé à l'intérieur ou au niveau du port universel respectif ; et
- un processeur ou un dispositif de commande (28).

6. Moniteur patient (20) selon l'une quelconque des revendications précédentes,
dans lequel l'unité de détection (28 ; 28A-28C) est configurée pour obtenir des informations sur le type de câble, dans lequel les informations sur le câble sont stockées :
dans une mémoire fournie au niveau du câble (12) ;
sous la forme d'une identification lisible de manière optique, fournie au niveau du câble (12) ; et/ou
sur une étiquette d'identification par radiofréquence, RFID, ou une étiquette de communication en champ proche, NFC, fournie dans ou au niveau du câble (12).

7. Moniteur patient (20) selon l'une quelconque des revendications précédentes,
dans lequel les deux ou plusieurs indicateurs optiques (22) sont configurés pour afficher des couleurs, des lettres et/ou des symboles graphiques différents.

8. Moniteur patient (20) selon l'une quelconque des revendications précédentes,
dans lequel les deux ou plusieurs indicateurs optiques (22) sont configurés pour indiquer si le type de câble d'un câble (12) connecté au port universel respectif (21) est pris en charge par le moniteur patient (20) et/ou le port universel (21) auquel le câble (12) est connecté.

9. Moniteur patient (20) selon l'une quelconque des revendications précédentes,
dans lequel les deux ou plusieurs indicateurs optiques (22) sont configurés pour indiquer :
un statut de mesure sur un sujet (10) ;
un état de transmission d'un signal de mesure ;
les paramètres d'une mesure ; et/ou
les paramètres d'une mesure sur un sujet (10) étant dans ou hors d'une plage valide des paramètres.

10. Moniteur patient (20) selon l'une quelconque des revendications précédentes,
dans lequel les deux ou plusieurs indicateurs optiques (22) sont configurables selon les préférences de l'utilisateur.

11. Moniteur patient (20) selon l'une quelconque des revendications précédentes,
comprenant en outre un ou plusieurs indicateurs optiques supplémentaires (22) par port universel (21).

12. Moniteur patient (20) selon l'une quelconque des revendications précédentes,
dans lequel un ou plusieurs indicateurs optiques (22) sont disposés en groupe au niveau et/ou à côté d'un groupe d'un ou plusieurs ports universels (21).

13. Moniteur patient (20) selon l'une quelconque des revendications précédentes,
dans lequel un ou plusieurs indicateurs optiques (22) sont des écrans, notamment des écrans à encre électronique.

14. Moniteur patient (20) selon l'une quelconque des revendications précédentes,
dans lequel un ou plusieurs ports universels (21) sont agencés sur ou au niveau d'un module enfichable (24) configuré pour être branché sur le moniteur patient (20).
